(19) **Europäisches Patentamt / European Patent Office / Office européen des brevets**

(11) **EP 4 471 788 A1**

(12) **EUROPEAN PATENT APPLICATION**
published in accordance with Art. 153(4) EPC

(43) Date of publication:
04.12.2024 Bulletin 2024/49

(21) Application number: 23747035.6

(22) Date of filing: 26.01.2023

(51) International Patent Classification (IPC):
*G16C 20/10* (2019.01)

(52) Cooperative Patent Classification (CPC):
**G16C 20/10**

(86) International application number:
**PCT/JP2023/002443**

(87) International publication number:
**WO 2023/145825 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 28.01.2022 JP 2022011509

(71) Applicant: **National University Corporation Hokkaido University**
**Hokkaido 060-0808 (JP)**

(72) Inventor: **MAEDA, Satoshi**
**Sapporo-shi, Hokkaido 060-0808 (JP)**

(74) Representative: **Stöckeler, Ferdinand et al**
**Schoppe, Zimmermann, Stöckeler**
**Zinkler, Schenk & Partner mbB**
**Patentanwälte**
**Radlkoferstrasse 2**
**81373 München (DE)**

(54) **REACTION-PATH SEARCH PROGRAM, REACTION-PATH SEARCH SYSTEM, AND REACTION-PATH SEARCH METHOD**

(57)     Increase in computational cost in accordance with increase in the number of atoms can be easily suppressed in search for a chemical reaction path.

For each of multiple fragment pairs formed of atoms in a structure that is the target of reaction path search, either a second differential coefficient b or a third differential coefficient a of $E(Q)$ at $Q=Q_0$, which corresponds to a first equilibrium state that is one of equilibrium states, where $E(Q)$ takes a local minimum value on the potential energy surface (PES) of the structure, is calculated. Subsequently, reaction path search in transition from the first equilibrium state to a second equilibrium state that is another of the equilibrium states is performed for a fragment pair with a high priority based on the magnitude of a and b among fragment pairs, prior to the other fragment pairs with lower priority.

FIG.1

**Description**

[Technical Field]

The present teaching relates to a reaction-path search program, a reaction-path search system, and a reaction-path search method for searching for a chemical reaction path.

[Background Art]

One approach for searching for a chemical reaction path for a given structure is an artificial force-induced reaction (AFIR) method described in Non-Patent Literature 1. The AFIR method is a method for inducing deformation of a structure by applying a force to a fragment pair formed of multiple atoms in the structure, so as to transition the structure to another structure. At this time, a corresponding reaction path is obtained from coordinate changes that the system follows during the transition. As this operation is systematically applied to various fragment pairs in the structure, reaction paths to various other structures are automatically searched for.

[Citation List]

[Non-Patent Literatures]

[Non-Patent Literature 1] Satoshi MAEDA and Yu HARABUCHI, "Exploring paths of chemical transformations in molecular and periodic systems: An approach utilizing force", Wiley Interdisciplinary Reviews: Computational Molecular Science, 11, e1538

[Summary]

[Technical Problem]

The number of fragment pairs that should be taken into consideration in a system formed of a given structure increases in proportion to the square of the number of atoms N in the system. The search of a reaction path by the AFIR method is performed for each fragment pair. On this account, when the number of atoms in a structure that is a target of the search increases, the number of combinations of fragment pairs which should be computed drastically increases, with the result that the computational cost required for searching for reaction paths may become enormous.

An object of the present teaching is to provide a reaction-path search program, reaction-path search system, and reaction-path search method that can easily suppress an increase in computational cost associated with an increase in the number of atoms.

[Solution to Problem]

A reaction-path search program of an aspect of the present teaching is for causing at least one computer to function as a reaction-path search system which is configured to calculate a reaction path in a structure formed of multiple atoms as a change in a structure represented by a positional relationship of the multiple atoms, and the reaction-path search system includes: a structural change calculation unit which is configured to calculate a change in the structure in which a result of a function $F^{AFIR}(Q)$ of an equation 1 calculated based on an equation 2 is at the minimum, where, in regard to a fragment pair of a fragment A composed of $N_1$ ($N_1$ is a natural number) atoms sampled from the multiple atoms and a fragment B composed of $N_2$ ($N_2$ is a natural number) atoms sampled from the multiple atoms and different from the atoms of the fragment A, potential energy at a geometric parameter Q indicating positions of the multiple atoms is $E(Q)$, distance between s-th (s is a natural number satisfying $s \leq N_1$) atom belonging to the fragment A and a t-th (t is a natural number satisfying $t \leq N_2$) atom belonging to the fragment B is $r_{st}$, $R_s$ and $R_t$ are covenant radii of the s-th atom and the t-th atom, respectively, $\rho$ is either +1 or -1, and p and $\alpha$ are constants; a differential coefficient calculation unit which is configured to calculate, for each of the fragment pairs formed of different combinations of atoms, at least one of a second differential coefficient b or a third differential coefficient a of $E(Q)$ at the positions of the multiple atoms, which correspond to a first equilibrium state that is one of equilibrium states where $E(Q)$ takes a local minimum value; and an equilibrium state change calculation unit which is configured to cause the structural change calculation unit to calculate the change in the structure in transition from the first equilibrium state to a second equilibrium state that is one of the equilibrium states, prior to the other fragment pairs with lower priority than the fragment pair with higher priority based on the magnitude of a and b calculated by the differential coefficient calculation unit among the fragment pairs.

(Equation 1)

$$F^{\mathrm{AFIR}}(\boldsymbol{Q}) = E(\boldsymbol{Q}) + \rho\alpha\frac{\sum_{s\in A}\sum_{t\in B}\omega_{st}r_{st}}{\sum_{s\in A}\sum_{t\in B}\omega_{st}}$$

(Equation 2)

$$\omega_{st} = \left[\frac{R_s + R_t}{r_{st}}\right]^p$$

According to another aspect of the present teaching, a reaction-path search system is a system for calculating a reaction path in a structure formed of multiple atoms as a change in a structure represented by a positional relationship of the multiple atoms, and includes: a structural change calculation unit which is configured to calculate a change in the structure in which a result of a function $F^{\mathrm{AFIR}}(Q)$ of an equation 1 calculated based on an equation 2 is at the minimum, where, in regard to a fragment pair of a fragment A composed of $N_1$ ($N_1$ is a natural number) atoms sampled from the multiple atoms and a fragment B composed of $N_2$ ($N_2$ is a natural number) atoms sampled from the multiple atoms and different from the atoms of the fragment A, potential energy at a geometric parameter Q indicating positions of the multiple atoms is $E(Q)$, distance between s-th (s is a natural number satisfying $s \leq N_1$) atom belonging to the fragment A and a t-th (t is a natural number satisfying $t \leq N_2$) atom belonging to the fragment B is $r_{st}$, $R_s$ and $R_t$ are covenant radii of the s-th atom and the t-th atom, respectively, $\rho$ is either +1 or -1, and p and $\alpha$ are constants; a differential coefficient calculation unit which is configured to calculate, for each of the fragment pairs formed of different combinations of atoms, at least one of a second differential coefficient b or a third differential coefficient a of $E(Q)$ at the positions of the multiple atoms, which correspond to a first equilibrium state that is one of equilibrium states where $E(Q)$ takes a local minimum value; and an equilibrium state change calculation unit which is configured to cause the structural change calculation unit to calculate the change in the structure in transition from the first equilibrium state to a second equilibrium state that is another one of the equilibrium states, prior to the other fragment pairs with lower priority than the fragment pair with higher priority based on the magnitude of a and b calculated by the differential coefficient calculation unit among the fragment pairs.

According to another aspect of the present teaching, a reaction-path search method is a method for calculating a reaction path in a structure formed of multiple atoms as a change in a structure represented by a positional relationship of the multiple atoms, and includes: a structural change calculation step of calculating a change in the structure in which a result of a function $F^{\mathrm{AFIR}}(Q)$ of an equation +1 calculated based on an equation 2 is at the minimum, where, in regard to a fragment pair of a fragment A composed of $N_1$ ($N_1$ is a natural number) atoms sampled from the multiple atoms and a fragment B composed of $N_2$ ($N_2$ is a natural number) atoms sampled from the multiple atoms and different from the atoms of the fragment A, potential energy at a geometric parameter Q indicating positions of the multiple atoms is $E(Q)$, distance between s-th (s is a natural number satisfying $s \leq N_1$) atom belonging to the fragment A and a t-th (t is a natural number satisfying $t \leq N_2$) atom belonging to the fragment B is $r_{st}$, $R_s$ and $R_t$ are covenant radii of the s-th atom and the t-th atom, respectively, $\rho$ is either +1 or -1, and p and $\alpha$ are constants; a differential coefficient calculation step of calculating, for each of the fragment pairs formed of different combinations of atoms, at least one of a second differential coefficient b or a third differential coefficient a of $E(Q)$ at the positions of the multiple atoms, which correspond to a first equilibrium state that is one of equilibrium states where $E(Q)$ takes a local minimum value; and an equilibrium state change calculation step of calculating, by the structural change calculation step, the change in the structure in transition from the first equilibrium state to a second equilibrium state that is another one of the equilibrium states, prior to the other fragment pairs with lower priority than the fragment pair with higher priority based on the magnitude of a and b calculated by the differential coefficient calculation unit among the fragment pairs.

According to the reaction-path search program, the reaction-path search system, and the reaction-path search method of the present teaching, a reaction path in a structure formed of multiple atoms is computed as a change in a structure represented by the positional relationship of the atoms. At this time, for a fragment pair with a high priority based on the magnitude of at least one of the second-order differential coefficient b or the third-order differential coefficient a of $E(Q)$ at the positions of the atoms corresponding to the first equilibrium state, the change in the structure is computed prior to a fragment pair with a low priority. For example, when the second-order differential coefficient b is small, it is indicated that local increase in $E(Q)$ tends to be small. Therefore, for example, if a fragment pair with small b is prioritized, it is possible to obtain a path with a small increase in $E(Q)$ at around the first equilibrium state. Furthermore, when the third-order differential coefficient a is small, it is indicated that a change from a concave curve to a convex curve tends to occur at $E(Q)$. Therefore, as another example, it is expected that a transition state will be more easily found at around the first equilibrium state when a fragment pair with small a is prioritized. Therefore, by prioritizing the calculation of certain fragment pairs based on the magnitude of at least one of the second-order differential coefficient b or the third-order differential coefficient a of E(Q) over the other fragment pairs, a target reaction path is more likely to be obtained without incurring much computational cost. On this account, increase in the computational cost in accordance with the increase in the number of atoms tends to be suppressed.

In addition, in the present teaching, preferably, the reaction path search system further includes a state selector which is configured to select one of N equilibrium states (N is a natural number of 2 or more) formed of all second equilibrium states obtained based on past calculation results by the equilibrium state change calculation unit and an initial state as the first equilibrium state, and the equilibrium state change calculation unit repeatedly causes the structural change calculation unit to calculate the change in the structure regarding the fragment pair with respect to the transition from the first equilibrium state selected by the state selector to the second equilibrium state. According to this arrangement, one of the already acquired equilibrium states is referred to as the first equilibrium state, and the calculation of the structural change from the first equilibrium state to the second equilibrium state is repeated, i.e., the acquisition of a reaction path is repeated. By this, it is possible to acquire a network formed of acquired equilibrium states composed of an initial state and a second equilibrium state obtained in the past, as well as reaction paths connecting these equilibrium states.

In addition to the above, in the present teaching, the reaction-path search system preferably includes a rate constant contraction unit which is configured to obtain a rate constant matrix, which is formed of l*l rate constants regarding transition between l (l is a natural number satisfying l<N) super states expressed as a weighted sum of the N equilibrium states, from a rate constant matrix of N rows and N columns, which is formed of N*N rate constants regarding transition between the N equilibrium states, by performing contracting of the rate constant matrix m times (m is a natural number satisfying m=N-l) based on an RCMC method, and the state selector selects one of the N equilibrium states as the first equilibrium state for each of m=1, 2, ..., M (M is a natural number satisfying M<N) based on a result of acquisition of a contracted rate constant matrix by the rate constant contraction unit so that, the larger $p_i(m)$ calculated based on an equation 3 and $\Lambda_i$ calculated based on an equation 4 are, the more likely $EQ_i$ is selected, when each of the N equilibrium states is $EQ_i$ (i is a natural number equal to or smaller than N), each of the l super states is $SS_j$ (j is a natural number equal to or smaller than l), population of $EQ_i$ after performing the contraction m times is $p_i(m)$, population of $SS_j$ after performing the contraction m times is $Q_j(m)$, contribution of $EQ_i$ to $SS_j$ after performing the contraction m times is $\chi_{ji}(m)$, relative Gibbs energy of $EQ_i$ is $\Delta G_i$, a gas constant is R, and a model temperature parameter is $T_R$. According to this arrangement, an equilibrium state with a large $\Lambda_i$ representing a traffic amount of a reaction path is more likely to be selected in the computation of the reaction path.

(Equation 3)

$$p_i^{(m)} = \sum_j^{\text{all SSs}} Q_j^{(m)} \frac{\chi_{ji}^{(m)} \, exp\left[\frac{-\Delta G_i}{RT_R}\right]}{\sum_k^{\text{all EQs}} \chi_{jk}^{(m)} \, exp\left[\frac{-\Delta G_k}{RT_R}\right]}$$

(Equation 4)

$$\Lambda_i = \sum_{m=1}^{M} \left| p_i^{(m)} - p_i^{(m-1)} \right|$$

In addition, in the present teaching, preferably, the state selector selects one of the N equilibrium states as the first equilibrium state so that the likeliness of selection of $EQ_i$ increases as $\Lambda_i$ increases, the likeliness of selection of $EQ_i$ increases as the number of times $n_i$ of calculation of the change in the structure by the structural change calculation unit while $EQ_i$ is set as the first equilibrium state decreases, and the larger the total number of times $n_{all}$ of calculation of the change in the structure by the structural change calculation unit for the N equilibrium states is, the less likely a diference in $\Lambda_i$ is reflected in a difference in the likeliness of selection of the equilibrium state. According to this, when $n_{all}$ is relatively small, an equilibrium state having large $\Lambda_i$ and small $n_i$ is likely to be selected as a target of computation. On this account, an equilibrium state with a large $\Lambda_i$ (i.e., a large traffic amount) is likely to be selected in a suitable manner, until $n_{all}$ becomes sufficiently large after the start of the computation of the change in the structure. When $n_{all}$ becomes relatively large, a difference in $\Lambda_i$ becomes less apparent in the easiness of the selection of the equilibrium state, accordingly. On this account, an equilibrium state with a small traffic amount becomes to be selected. In this way, according to the arrangement above, selection of an equilibrium state is performed suitably depending on the priority that changes in accordance with the advance of the computation.

In addition, in the present teaching, when the total number of times of calculation of the change in the structure by the structural change calculation unit for the N equilibrium states is $n_{all}$, the number of times of calculation of the change in the structure by the structural change calculation unit while $EQ_i$ is set as the first equilibrium state is $n_i$, $\xi_i$ is a real number not smaller than 0 and not larger than 1, and each of $\alpha$ and $\beta$ is a real number not smaller than 0 and not larger than 1, the state selector selects $EQ_i$ with which $v_i$ in an equation 5 is the largest, as the first equilibrium state. $v_i$ obtained by the equation 5 increases as $\Lambda_i$ increases. When $n_{all}$ is relatively small, $v_i$ increases as $n_i$ decreases. However, when $n_{all}$ is relatively large, a difference in $\Lambda_i$ becomes less apparent in a difference in $v_i$. On this account, as $EQ_i$ with the largest $v_i$ is selected, selection of an equilibrium state is performed suitably depending on the priority that changes in accordance with the advance of the computation.

(Equation 5)

$$v_i = \xi_i \left( \Lambda_i + \alpha^{\frac{n_i}{\log(n_{\text{all}})}} \right) \beta^{\frac{n_i}{\log(n_{\text{all}})}}$$

[Brief Description of Drawings]

FIG. 1 is a conceptual diagram of a fragment pair formed of plural atoms in an AFIR method employed in an embodiment of the present teaching.
FIG. 2 is a conceptual diagram showing the network of reaction paths obtained based on the AFIR method used in one embodiment of the present teaching.
FIG. 3 is a block diagram illustrating the functional configuration of a reaction-path search system according to one embodiment of the present teaching.
FIG. 4 is a graph conceptually showing changes in PES when the relative positions of atoms in a 0-th layer of the fragment pairs in FIG. 1 are changed along a straight line passing through these atoms, in regard to the vicinity of the equilibrium state $EQ_0$.
FIG. 5 is a flowchart showing a series of processes performed by the reaction-path search system shown in FIG. 3.

[Description of Embodiments]

The following will describe a reaction-path search system 1 according to one embodiment of the present teaching, with reference to figures. The reaction-path search system 1 is a system configured to search for a reaction path in a structure such as a molecule formed of multiple atoms. The reaction-path search system 1 searches for a reaction path based on an artificial force-induced reaction method (AFIR method), specifically a single-component AFIR method (SC-AFIR method). To begin with, an overview of a method for searching a reaction path based on the SC-AFIR method will be described. Then details such as the arrangement and functions of the reaction-path search system 1 will be given.

[Overview of Reaction-Path Search Method]

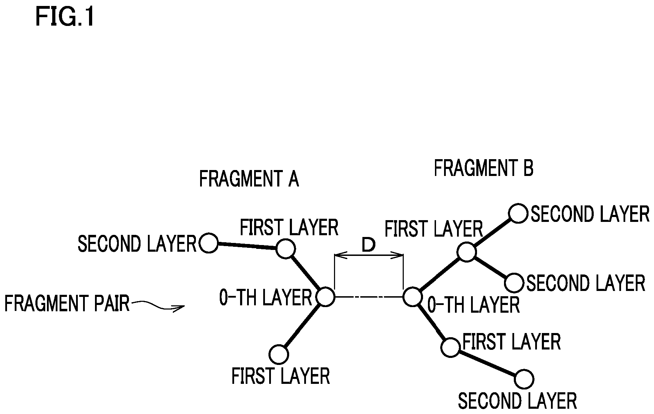

The AFIR method is a method for inducing structural transformation by applying a force between a fragment pair formed of fragments A and B, as shown in FIG. 1, by using an AFIR function $F^{AFIR}$ shown in the following equations 1 and 2.

(Equation 1)

$$F^{\text{AFIR}}(\boldsymbol{Q}) = E(\boldsymbol{Q}) + \rho\alpha \frac{\sum_{s \in A} \sum_{t \in B} \omega_{st} r_{st}}{\sum_{s \in A} \sum_{t \in B} \omega_{st}}$$

(Equation 2)

$$\omega_{st} = \left[ \frac{R_s + R_t}{r_{st}} \right]^p$$

The fragment A is a fragment of a structure formed of $N_1$ ($N_1$ is a natural number) atoms in the above-described structure. The fragment B is a fragment of a structure formed of other $N_2$ ($N_2$ is a natural number) atoms in the above-described structure. E(Q) corresponds to a potential energy surface (PES) in geometric parameters Q indicating the positions of the atoms in the above-described structure. $r_{st}$ indicates the distance between an s-th atom (s is a natural number satisfying $s{\leq}N_1$) belonging to the fragment A and a t-th atom (t is a natural number satisfying $t{\leq}N_2$) belonging to the fragment B. $R_s$ and $R_t$ are covenant radii of the s-th atom and the t-th atom, respectively, $\rho$ is set at either +1 or - 1. p is suitably selected from a range of 4 to 8, inclusive (e.g., p=6). $\alpha$ which indicates the magnitude of a force applied between fragments is a constant that is based on an equation 1-1 below. As $R_0$ and $\varepsilon$ in the equation 1-1, Ar-Ar Lennard-Jones parameters, i.e., $3.8164*10^{-10}$m and 1.0061kJ/mol are employed, respectively, $\alpha$ that is expressed by the equation 1-1 corresponds to an average force received at the time of movement from the least point to the turning point, when paired Ar and Ar directly collide with each other with a collision energy $\gamma$. Therefore $\gamma$ is termed a model collision energy parameter and corresponds to a rough upper limit of a barrier with which the force in the AFIR method can cope. $\gamma$ may be optionally set by a user.

(Equation 1-1)

$$\alpha = \frac{\gamma}{\left[ 2^{-\frac{1}{6}} - \left( 1 + \sqrt{1 + \frac{\gamma}{\varepsilon}} \right)^{-\frac{1}{6}} \right] R_0}$$

A reaction path is a path obtained by minimizing an AFIR function by using a standard quasi-Newton method (see Non-Patent Literature 1). This path is termed an AFIR path. Along the AFIR path, an approximate equilibrium (EQ) structure and a transition state (TS) structure are obtained as a structure with which the AFIR function is at the local minimum and a structure with which the AFIR function is at the local maximum, respectively. These structures can be used as initial guess of actual EQ and TS. However, the maximum value of these energies may be significantly different from the actual TS. This may occur especially when $\gamma$ is too large. Even in such a case, an overlook of the TS is avoided by performing a relaxation computation of the AFIR path. For this purpose, any double-ended method can be used to optimize the entire path point. As a method for this, a Local Plane Update (LUP) method is adopted (J. Chem. Phys. 1991, 94, 751). According to an implementation example using the LUP method, path points are evenly distributed along a specific path, and move to low-energy points within a hyperplane perpendicular to the tangent of the path. A path obtained by this is termed an LUP path. The two end points and all local maximum values are directly optimized to the minimum value and the maximum value. An approximate EQ and an approximate TS on the AFIR path or the LUP path are optimized to the actual EQ and TS by using a standard quasi-Newton method. Subsequently, starting from all the acquired TS, intrinsic reaction coordinates (IRC) are calculated. Lastly, for all the acquired EQ and TS, a normal mode analysis is performed.

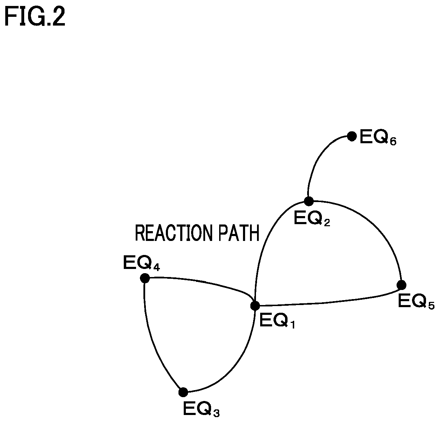

According to an SC-AFIR method, an AFIR path is computed from EQ (J. Comput. Chem. 2014, 35, 166). Structures corresponding to EQ include molecules, molecular complexes, organic metal complexes, clusters, surface adsorption structures, and crystal structures. By minimizing an AFIR function of a fragment pair constituted by various fragments A and B, various AFIR paths are obtained from a single EQ. In a structure corresponding to each EQ, a fragment pair is systematically defined by focusing on an atom pair. In other words, as shown in FIG. 1, in the fragment A and the fragment B, two atoms are provided as 0-th-layer atoms. Next, the atoms in the first layer connected to the atoms in the 0-th layer and the atoms in the second layer connected to the atoms in the first layer are added to the fragments A and B. Among the atoms in the first and second layers in each fragment, an atom whose distance to the atoms in the other fragment is shorter than the distance between the 0-th-layer atoms is removed from each fragment. A series of fragment pairs are generated by applying this procedure to all atomic pairs, excluding those with very long distances. Subsequently, an AFIR path of each fragment pair is computed. If, hypothetically, AFIR paths are computed for all fragment pairs, and let $N_{atoms}$ be the number of atoms, the cost required to complete the calculation around a single EQ scales with $N_{atoms}^2$. As an AFIR path is computed with an obtained EQ as a start point and acquisition of new EQ and paths between EQs is repeated, a reaction path network that is constituted by N EQs (N is a natural number equal to or more than 2) is obtained as shown in FIG. 2. Hereinafter, in the reaction path network constituted by N EQs, an i-th (i is a natural number equal to or less than N) EQ may be termed $EQ_i$. Especially, $EQ_1$ indicates a reactant as an initial structure. FIG. 2 shows an example of a reaction path network when N=6.

Now, the following will describe a kinetic-based navigation that is employed to reduce the computational cost of reaction paths. It has been known that the number of EQs exponentially increases with the number of atoms $N_{atoms}$. On this account, unless the $N_{atoms}$ is small, it is difficult to apply the above-described computation procedure based on the SC-AFIR method to all EQs. On the other hand, the number of EQs that can be accessed dynamically under calm experimental conditions is limited. Therefore, the cost for searching for EQs that are kinetically inaccessible is eliminated by determining whether EQ is accessible kinetically under specific experimental conditions by using a rate constant matrix contraction (RCMC) method which is a kinetic approach (Chem. Lett. 2019, 48, 47).

The RCMC method makes it possible to perform a kinetic analysis considering the entire reaction path network formed of N EQs by repeating an operation called contraction. From N*N rate constants related to the transition between N EQs, a rate constant matrix of N rows and N columns is obtained. In the RCMC method, a smaller rate constant matrix of n rows and n columns (n=N-M) is obtained through M contractions. The rate constant matrix of n rows and n columns after M contractions represents a rate constant for transition between n super states (SS). SS is expressed as the weighted sum of all EQs. The size of the rate constant matrix decreases by one after each contraction, and based on a quasi-constant-state approximation and a Boltzmann distribution preservation condition, all nondiagonal elements of the rate constant matrix are updated. The nondiagonal elements of the rate constant matrix obtained by the contraction correspond to a rate constant for transition from one SS to another SS. The contraction is applied to all states in each of which the lifetime is shorter than a threshold $t_{MAX}$ that can be optionally set by a user. As a result of M contractions, n SSs that mutually transition with a time scale longer than $t_{MAX}$ are obtained. The RCMC method will be detailed later.

The population of $EQ_i$ after m contractions is $p_i(m)$. This population indicates the existence probability of the $EQ_i$ at a given time. In kinetic-based navigation, a user needs to provide an initial population with experimental conditions being taken into consideration. For example, when $EQ_1$ is a reactant, the initial population of $EQ_1$ is set at $1(p_1(0)=1)$, whereas the population of each of all other EQs is set at $0(p_{i\neq1}(0)=0)$. Next, an index $\Lambda_i$, i.e., a so-called traffic amount is computed for all EQi based on equations 3 and 4.

(Equation 3)

$$p_i^{(m)} = \sum_{j}^{\text{all SSs}} Q_j^{(m)} \frac{\chi_{ji}^{(m)} exp\left[\frac{-\Delta G_i}{RT_R}\right]}{\sum_k^{\text{all EQs}} \chi_{jk}^{(m)} exp\left[\frac{-\Delta G_k}{RT_R}\right]}$$

(Equation 4)

$$\Lambda_i = \sum_{m=1}^{M} \left| p_i^{(m)} - p_i^{(m-1)} \right|$$

In the equations 3 and 4, $Q^{(m)}_j$ indicates the population of $SS_j$ after m contractions, provided that each of n SSs is $SS_j$ (j is a natural number satisfying j≤n). $\chi^{(m)}_{ji}$ indicates the contribution of $EQ_i$ to $SS_j$ after m contractions. $\Delta G_i$ indicates a relative Gibbs energy of $EQ_i$ obtained by a normal mode analysis based on a quantum chemical computation. R is a gas constant and $T_R$ is a model temperature parameter. The equation 4 indicates the total of population inflow to $EQ_i$ and population outflow from $EQ_i$ in the $t_{MAX}$. For example, in a three-state system constituted by a reactant EQ, an intermediate EQ sufficiently more stable than the reactant EQ, and a product EQ sufficiently more stable than the intermediate EQ, when a path from the reactant EQ to the product EQ is open only when the path passes through the intermediate EQ as an experimental condition, the reactant EQ and the product EQ are $\Lambda_i$=1.0, and the intermediate EQ is $\Lambda_i$=2.0. On the other hand, when access to $EQ_i$ is not possible due to the experimental condition, $\Lambda_i$ is zero. In the present embodiment, one of N EQs is selected by using such a $\Lambda_i$ value, as described below. For the selected EQ, an AFIR path is computed. The AFIR path is computed for a fragment pair that is suitably selected from fragment pairs that have not been processed yet. $T_R$ determines the frequency of selection of a high-energy EQ from EQs having $\Lambda_i$ which is not zero. $T_R$ is typically set at a high value, e.g., 3000 K.

[System Details]

The reaction-path search system 1 includes hardware including one or more computers and software including a program that causes the hardware to function so that various functions of the reaction-path search system 1 are realized. Each computer is composed of memory devices such as a CPU (Central Processing Unit), a ROM (Read-Only Memory), and a RAM (Random Access Memory), and various interfaces such as an input/output interface. In addition, an input device configured to receive a user input, an output device such as a display, and a storage device such as a hard disk may be connected to the computer as external devices or may be mounted on the computer as internal devices. The software is constituted by, for example, program data recorded on storage devices such as a memory device and a hard disk. The hardware such as a computer performs various information processes such as an arithmetic process and an input/output process, under the control by the software. Various functions of the reaction-path search system 1, which will be described below, are realized by the functions of the hardware based on the software.

When the hardware includes plural computers, the computers may cooperate to execute the above-described processes related to the functions. The cooperation may be done in various manners. For example, computers may be connected to each other through a communication network such as the Internet, and the computers may exchange necessary information with each other and performs a necessary arithmetic process in a sharing manner. Alternatively, one computer may be in charge of a user interface function, and another computer may perform an arithmetic process based on input information that is input to the one computer by a user and is transmitted through the communication network.

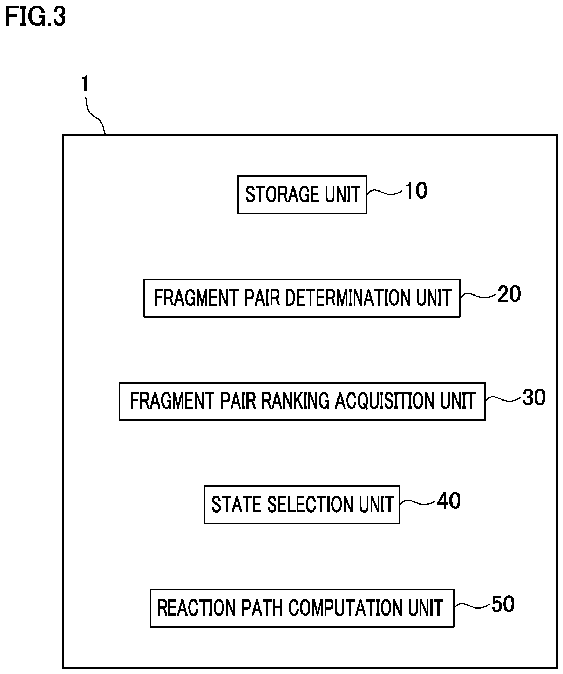

The reaction-path search system 1 includes a storage unit 10, a fragment pair determination unit 20, a fragment pair ranking acquisition unit 30, a state selection unit 40, and a reaction path computation unit 50, as shown in FIG. 3. The storage unit 10 stores data such as a list of acquired EQs, structural data indicating the coordinates of atoms, fragment data indicating combinations of atoms in fragments A and B, and search count data. The list of acquired EQs is composed of $EQ_1$ corresponding to a reactant and another EQ ($EQ_{i \neq 1}$) acquired by a reaction path search. The structural data includes data that indicates the coordinates of atoms for each of EQs included in the list of acquired EQs. The coordinates of atoms represent the types and positions of the atoms that make up the structure. The position of an atom is indicated, for example, by the three-dimensional position coordinates of the atom (e.g., coordinates on a Cartesian coordinate system). The fragment data is data indicating combinations of atoms in a fragment pair (fragments A and B) set in each EQ, for the list of acquired EQs. The storage unit 10 stores the fragment data in association with an EQ. If a ranking (priority in the present teaching) has been obtained for a fragment pair, the fragment data includes data indicating the ranking of that fragment pair. A high position in the ranking indicates a high priority in the reaction path search. The search count data indicates the number of times $n_i$ of execution of the reaction path search starting from $EQ_i$, for each $EQ_i$ (i=1, 2, ...) included in an EQ list that has already been obtained. The fragment data further includes data indicating whether the reaction path search has already been executed for each fragment pair. By default, the data indicates that the reaction path search has not been executed. In addition, the storage unit 10 stores data indicating various numerical conditions used by each part of the reaction-path search system 1 when performing a calculation described below.

The fragment pair determination unit 20 extracts combinations of atoms that constitute the fragments A and B from all atoms that make up the structure, based on the above-described method according to the SC-AFIR method. Fragment data indicating the extraction result is stored in storage unit 10. The fragment pair determination unit 20 generates a combination of atoms that compose a fragment pair for each $EQ_i$.

The fragment pair ranking acquisition unit 30 refers to the fragment data in the storage unit 10 and grasps an EQ (hereinafter, target EQ) for which the ranking of the fragment pair has not been obtained, from the list of acquired EQs. For each target EQ, the ranking is obtained in a manner described below.

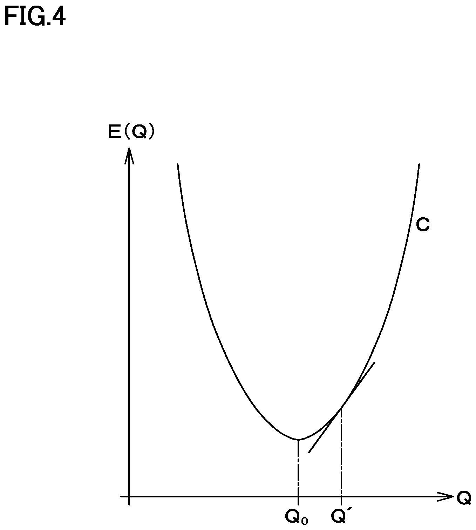

First, the fragment pair ranking acquisition unit 30 determines a second-order differential coefficient b and a third-order differential coefficient a of E(Q) at $Q=Q_0$ for each fragment pair determined by the fragment pair determination unit 20 with respect to the target EQ. Here, E(Q) represents PES as described above. Qo indicates the position of an atom in a structure corresponding to the target EQ. The second-order differential coefficient b and the third-order differential coefficient a are calculated based on, for example, the slope of the tangent line to a curve C at $Q=Q_0$ and Q=Q' shown in FIG. 4 as well as the value of E(Q). Q' ($=Q_0+\Delta Q$) represents the position of an atom in the structure after at least one of the paired 0-th-layer atoms is slightly displaced relative to the other along a straight line connecting the 0-th-layer atoms, so that the distance between the 0-th-layer atoms changes from D to $D+\Delta D$ (e.g., $\Delta D$=0.05 angstrom), where D is the distance between the paired 0-th-layer atoms in the fragment pair when $Q=Q_0$. The curve C is a curve extending along PES that shows changes in E(Q) when the relative positions of the paired 0-th-layer atoms are varied along the above-mentioned straight line. The fragment pair ranking acquisition unit 30 optimizes Q' or $\Delta Q$ to satisfy the following condition. The condition is, in regard to a distance matrix in which distances between all atoms are the elements, to allow matrix elements excluding an element corresponding to between the 0-th-layer atoms in the distance matrix when Q=Q' to reproduce matrix elements excluding an element corresponding to between the 0-th-layer atoms in the distance matrix when $Q=Q_0$ as good as possible, while fixing the distance between the paired 0-th-layer atoms to $D+\Delta D$. With the assumption that E(Q) is represented by a cubic function $f(x)=a_3 {}^* x^3 + a_2 {}^* x^2 + a_1 {}^* x + a_0$, it is possible to obtain each coefficient of f(x) by solving four simultaneous equations made of four equations $f(x(Q_0))=E(Q_0)$, $f(x(Q'))=E(Q')$, $f'(x(Q_0))=0$, and f'(x(Q'))=(inclination of tangent l of curve C at Q=Q') for $a_0$, $a_1$, $a_2$, and $a_3$. In this regard, x represents the coordinate axis along a straight line connecting paired 0-th-layer atoms, f'(x) represents a first derivative (df/dx) of f(x), and f''(x) represents a second derivative ($d^2f/dx^2$) of f(x). Furthermore, f(x(Q)) and f'(x(Q)) indicate values of f(x) and f'(x) at x corresponding to the position Q. By using f(x) in which the calculated $a_0$, $a_1$, $a_2$, and as are substituted, a and b are obtained with b=f'' ($Q_0$) and a=f'''($Q_0$). It is noted that f''' (x) is a third derivative ($d^3f/dx^3$) of f(x). The function and step of calculating the second-order differential coefficient b and the third-order differential coefficient a by the fragment pair ranking acquisition unit 30 correspond to a function and differential coefficient calculation step in a differential coefficient calculation unit of the present teaching.

The fragment pair ranking acquisition unit 30 determines the ranking of fragment pairs based on one of equations 4-1 to 4-4 represented by at least one of the second-order differential coefficient b or the third-order differential coefficient a obtained as described above. When one of $\zeta^{\text{Second}}$, $\zeta^{\text{Third}}$, $\zeta^{\text{Scaled}}$, and $\zeta^{\text{Average}}$ is used is determined based on a user input made through an input device. Among $\zeta^{\text{Second}}$, $\zeta^{\text{Third}}$, $\zeta^{\text{Scaled}}$, and $\zeta^{\text{Average}}$, the selected one is denoted as $\zeta$. The fragment pair ranking acquisition unit 30 sets the ranking in such a way that, the smaller $\zeta$ of a fragment pair is, the higher the fragment pair is evaluated in the ranking. The acquired ranking is stored in storage unit 10.

(Equation 4-1)

$$\zeta^{\text{Second}} = b$$

(Equation 4-2)

$$\zeta^{\text{Third}} = a$$

(Equation 4-3)

$$\zeta^{\text{Scaled}} = b^{-\frac{3}{2}}a$$

(Equation 4-4)

$$\zeta^{\text{Average}} = b^{-\frac{3}{4}}a$$

A direction in which $\zeta^{\text{Second}}$ decreases is considered as a direction in which local increase of E(Q) is small. On this account, as a reaction path search is performed for a fragment pair having a small $\zeta^{\text{Second}}$, it is expected that a path in which increase of E(Q) at around EQ is small is obtained. In a direction in which $\zeta^{\text{Third}}$, it is expected that a change from a concave curve to a convex curve occurs and a transition state is found. In this regard, the height of the local maximum point on the cubic function is determined by a balance of a and b. $\zeta^{\text{Third}}$ is particularly small when E(Q) rapidly changes from a concave curve to a convex curve in the direction in which b increases. Therefore, it is expected that a path where a hard bond such as a chemical bonds is rearranged with a low barrier is obtained by performing reaction path search for a fragment pair with small $\zeta^{\text{Third}}$. On the other hand, the smaller $\zeta^{\text{Scaled}}$ is, the smaller the local maximum point on the cubic function is. Therefore, it is expected that a path with a low barrier such as internal rotation and hydrogen bond rearrangement is obtained by performing reaction path search for a fragment pair with small $\zeta^{\text{Scaled}}$. In many chemical reactions, events with low barrier such as conformational changes are repeated in short time scales such as nanoseconds and microseconds, achieving local thermal equilibrium. Rare events such as rearrangement of chemical bonds, which involve overcoming of high barriers, occur on a long time scale, such as seconds or hours. In multistep reactions, the overall reaction progresses through repeated local thermal equilibration and rare events. To reproduce such a situation, it is necessary to calculate both a fragment pair with a small $\zeta^{\text{Third}}$ and a fragment pair with a small $\zeta^{\text{Scaled}}$. Due to this, $\zeta^{\text{Average}}$ obtained by performing geometric mean of $\zeta^{\text{Third}}$ and $\zeta^{\text{Scaled}}$ is introduced. $\zeta^{\text{Average}}$ is small in a fragment pair with a small $\zeta^{\text{Third}}$ or $\zeta^{\text{Scaled}}$. Therefore, it is expected that various paths such as internal rotation, hydrogen bond rearrangement, and chemical bond rearrangement are obtained by performing reaction path search for a fragment pair with small $\zeta^{\text{Average}}$.

The state selection unit 40 (state selector in the present teaching) selects an EQ that serves as the starting point of reaction path search. Specifically, the state selection unit 40 calculates $\Lambda_i$ shown in the above-described equation 4, and calculates $v_i$ in an equation 5 described below. Furthermore, the state selection unit 40 selects an $EQ_i$ with the largest $v_i$ as the starting point of the next reaction path search. When calculating $\Lambda_i$, the state selection unit 40 performs a contraction operation of a rate constant matrix based on the RCMC method as described above. Such a function of the state selection unit 40 corresponds to a function of a rate constant contraction unit in the present teaching.

(Equation 5)

$$v_i = \xi_i \left( \Lambda_i + \alpha^{\frac{n_i}{\log(n_{\text{all}})}} \right) \beta^{\frac{n_i}{\log(n_{\text{all}})}}$$

$n_i$ is indicated by search count data stored in the storage unit 10. $n_{\text{all}}$ is the sum of $n_i$ of all $EQ_i$ up to that point. $\alpha$ and $\beta$ are real numbers greater than or equal to 0 and less than or equal to 1. For example, it is possible to set $\alpha=0.5$ and $\beta=0.5$. $\alpha$ plays a role in ensuring that even in $EQ_i$ with a small $\Lambda_i$, $EQ_i$ is selected multiple times. $\beta$ plays a role in making it less likely for

$EQ_i$ to be selected by terms that include $\alpha$. $\xi_i$ is a random real number between 0 and 1, inclusive. $\xi_i$ is set for each calculation of $v_i$ based on the equation 5.

The reaction path computation unit 50 performs reaction path search in which EQ selected by the state selection unit 40 is set as a starting point EQ (first equilibrium state of the present teaching). The reaction path computation unit 50 performs reaction path search for fragment pairs indicated by fragment data stored in the storage unit 10 in association with this EQ. In doing so, the reaction path computation unit 50 performs reaction path search for a fragment pair having a high ranking prior to a fragment pair having a low ranking, among the fragment pairs. In other words, reaction path search is performed for a fragment pair having a high ranking, before performing reaction path search for a fragment pair having a low ranking. For such fragment pairs for which the reaction path search has been performed, the fragment data stored in the storage unit 10 is updated to indicate that the reaction path search has already been performed.

In the reaction path search, as described above, calculation of AFIR and LUP paths, optimization of a transition state, and calculation of IRC are performed. As a result, a new reaction path with the EQ selected by the state selection unit 40 as a starting point is obtained. The reaction path computation unit 50 refers to the storage content of the storage unit 10, and in an EQ (second equilibrium state in the present teaching) through which an acquired reaction path passes or reaches, if there is a new EQ that is not included in the list of the acquired EQs, the new EQ is added to the list of acquired EQs indicated by the storage content of the storage unit 10. In addition, structural data indicating the coordinates of atoms corresponding to the new EQ is added to the storage content of the storage unit 10. Regarding this new EQ, the fragment pair determination unit 20 extracts combinations of atoms that constitute a fragment pair, and the fragment pair ranking acquisition unit 30 obtains the rankings for the fragment pair thus determined. A function and step of calculating an AFIR path by the reaction path computation unit 50 correspond to a function of a structural change calculation unit and a structural change calculation step of the present teaching. A function and step of the reaction path computation unit 50 calculating a reaction path corresponding to transition to another EQ by calculating an AFIR path using an EQ selected by the state selection unit 40 as a starting point correspond to a function of an equilibrium state change calculation unit and an equilibrium state change calculation step of the present teaching.

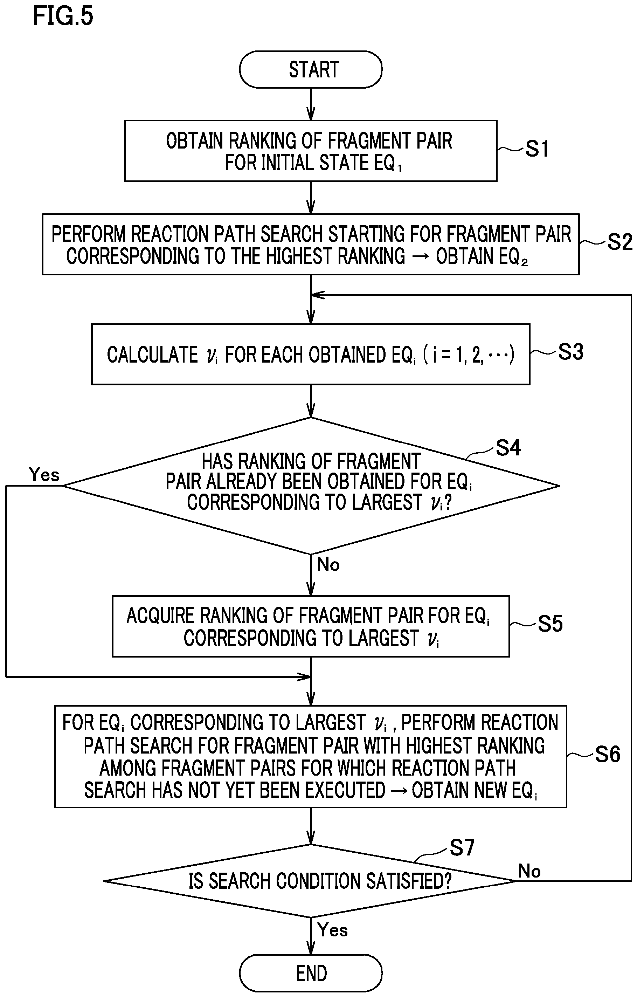

The following describes the sequence of the reaction path search method performed by the reaction path search system 1, with reference to FIG. 5. First, the fragment pair determination unit 20 and the fragment pair ranking acquisition unit 30 obtain a fragment pair and its ranking for an initial state $EQ_1$ given by the user (step S1). The structural data in the initial state $EQ_1$ is, for example, provided by importing a data file generated by the user into the system.

Subsequently, the reaction path computation unit 50 performs reaction path search starting from $EQ_1$ for a fragment pair corresponding to the highest ranking obtained in the step S1 (step S2). Based on a reaction path obtained by this step, $EQ_2$ that is a new EQ is added to the list of acquired EQs.

Subsequently, the state selection unit 40 calculates $v_i$ shown in the equation 5 for each $EQ_i$ (i=1, 2, ...) in the list of the acquired EQs (step S3). Subsequently, the fragment pair ranking acquisition unit 30 determines whether the ranking of the fragment pair has already been obtained for the $EQ_i$ corresponding to the largest $v_i$ calculated in the step S3 (step S4). This determination is performed by referring to the fragment data stored in the storage unit 10. When the fragment pair ranking acquisition unit 30 determines that the ranking has already been acquired (Yes in the step S4), the step S6 is executed. When the ranking has not been acquired yet, i.e., when the $EQ_i$ is the above-described target EQ (No in the step S4), the fragment pair ranking acquisition unit 30 acquires a ranking for a fragment pair related to $EQ_i$ corresponding to the largest one of $v_i$ calculated in the step S3 (step S5).

Thereafter, the reaction path computation unit 50 refers to the storage content of the storage unit 10 for $EQ_i$ corresponding to the largest $v_i$ calculated in the step S3, and performs reaction path search for a fragment pair with the highest ranking among the fragment pairs for which the reaction path search has not yet been executed (step S6). If a new EQ that is not in the list of acquired EQs is obtained by the reaction path search, the data related to that new EQ is added to the storage content of the storage unit 10.

Subsequently, the reaction-path search system 1 determines whether a search condition for searching for a reaction path is satisfied or not (step S7). The search condition is, for example, "in calculations of the past n AFIR paths, m EQs in the descending order from the largest traffic volume $\Lambda_i$ have not been updated." In this case, each of the natural numbers n and m may be set by the user. If it is determined that the required search condition is not set (No in the step S7), steps are executed from the step S3. If it is determined that the search condition is satisfied (Yes in the step S7), the reaction-path search system 1 terminates the sequence.

According to the reaction-path search system 1 of the present teaching described above, the reaction path search is performed for a fragment pair with a high priority based on the magnitude of at least one of the second-order differential coefficient b or the third-order differential coefficient a of E(Q) at the positions of the atoms corresponding to an equilibrium state EQ, prior to a fragment pair with a low priority. To be more specific, the ranking is set for a fragment pair based on $\zeta$ selected by the user from one of $\zeta^{Second}$, $\zeta^{Third}$, $\zeta^{Scaled}$, and $\zeta^{Average}$ in the above-described equations 4-1 to 4-4. The reaction path search is performed for the fragment pairs in the descending order of the ranking. When the reaction path search is preferentially performed for a fragment pair with a high ranking based on $\zeta^{Second}$, $\zeta^{Third}$, $\zeta^{Scaled}$, and $\zeta^{Average}$ as described above, it is easy to obtain a target reaction path, because, for example, it is easy to find a transition state, it is easy to find a path where a hard bond such as a chemical bonds is rearranged with a low barrier, it is easy to find a path with a low barrier such as internal rotation and hydrogen bond rearrangement, and it is easy to obtain various paths such as internal rotation, hydrogen bond rearrangement, and chemical bond rearrangement. This makes it possible to obtain a target reaction path without incurring much computational cost. On this account, increase in the computational cost in accordance with the increase in the number of atoms tends to be suppressed.

In addition, in the present embodiment, $EQ_i$ with the largest $\nu_i$ is selected, and the reaction path search is performed for that $EQ_i$. $\nu_i$ is arranged such that, as shown in the equation 5, when $n_{all}$ is relatively small, the value of $\alpha^{\{n_i/\log(n_{all})\}}$ in the equation 5 is small relative to $\Lambda_i$, and hence a difference in $\Lambda_i$ tends to be apparent. On this account, an equilibrium state with a large traffic amount tends to be suitably selected as a computation target. On the other hand, as $n_{all}$ becomes relatively large, the value of $\alpha^{\{n_i/\log(n_{all})\}}$ in the equation 5 becomes large relative to $\Lambda_i$. To put it differently, a difference in $\Lambda_i$ becomes less apparent in a difference in $\nu_i$. Therefore, when $n_{all}$ becomes relatively large, a difference in $\Lambda_i$ becomes less apparent in the easiness of the selection of the equilibrium state. On this account, an equilibrium state with a small traffic amount becomes to be selected. In this way, according to the arrangement above, an equilibrium state with a high priority is suitably selected in accordance with advance of the computation.

[Example]

The following describes an example of the present teaching. In this example, the reaction-path search method of the above-described embodiment shown in FIG. 5 was carried out in regard to a Strecker reaction, which is the most basic organic synthesis reaction, with some of the steps being changed. The Strecker reaction is a reaction in which ketone (or aldehyde), ammonia, and hydrogen cyanide react so that amino nitrile is generated. In this example, acetone was used as ketone. Furthermore, an extra water molecule was added to the system, taking into account the efficiency of proton transfer in an aqueous solution. The collision energy parameter $\gamma$ of a force applied by the SC-AFIR method was set to 500 kJ/mol. In order to prevent molecules from being excessively separated, a weak force of $\gamma=100/\{N(N-1)/2\}$kJ/mol was applied to all atom pairs. (N indicates the number of atoms included in the system.) To reduce computational complexity, as the above-described change, the optimization of the transition state after the computation of the LUP path and the computation of IRC were not performed. Instead, the energy local maximum point of the LUP path was used as an approximate transition state, and the LUP path was used as a path defining the connection between EQs. In the harmonic vibration analysis in the computation of Gibbs energy, the frequency of a mode having a vibration frequency of equal to or less than 50cm$^{-1}$ was set at 50cm$^{-1}$. An electronic state computation was performed by using Gaussian16 and at an RHF/SV level.

In the computation of $\nu_i$, the largest $\Lambda_i$ obtained by performing the RCMC method at three temperatures, 200K, 300K, and 400K, was used. When the $\Lambda_i$ was zero at all three temperatures, the corresponding $\nu_i$ was also forcibly set to zero. In addition, a reaction time was 1 day, air pressure was 1 atm, and $T_R$ was 3000K. The reaction path search was performed not only when fragment pairs to be targeted were selected with the preference based on the ranking assigned using $\zeta$Second, $\zeta$Third, $\zeta$Scaled, and $^$Average, but also when fragment pairs to be targeted were randomly selected. The search was terminated when a product was obtained, and the computational cost incurred up to that point was compared between the cases using $\zeta^{Second}$, $\zeta^{Third}$, $\zeta^{Scaled}$, and $\zeta^{Average}$. These computations used the same initial structure (structure of reactants) and the structure of the product. Table 1 shows a result of the comparison. Table 2 shows the x-coordinate, y-coordinate, and z-coordinate of each atom in the initial structure, and Table 3 shows the x-coordinate, y-coordinate, and z-coordinate of each atom in the structure of the product.

(Table 1)

| | $N^{gradient}$ | $N^{Hessian}$ | $N^{EQ}$ | $N^{path}$ |
|---|---|---|---|---|
| $\zeta^{Second}$ | 768763 | 20330 | 425 | 1884 |
| $\zeta^{Third}$ | 688830 | 19324 | 408 | 1795 |
| $\zeta^{Scaled}$ | 552532 | 15555 | 360 | 1438 |
| $\zeta^{Average}$ | 359092 | 10424 | 230 | 921 |
| Random | 805435 | 28082 | 498 | 2597 |

(Table 2)

| | x | y | z |
|---|---|---|---|
| O | 1.864801414878 | 0.897211313302 | 2.155175053733 |
| H | 2.762669262449 | 1.215652378929 | 2.055107305253 |
| H | 1.721988493373 | 0.427213696970 | 3.018944424716 |
| C | 1.167148346434 | 3.139463705830 | 4.066856326087 |

(continued)

|   | x | y | z |
|---|---|---|---|
| O | 2.008349957274 | 2.747340333995 | 4.868461507067 |
| C | 1.537245103343 | 4.019234453289 | 2.915168071723 |
| C | -0.268916332299 | 2.727346218186 | 4.194096773348 |
| H | 1.096980800012 | 3.641209907673 | 1.993046549869 |
| H | -0.485721047841 | 2.431218636779 | 5.218956480030 |
| H | 2.619751560429 | 4.064044356982 | 2.819032026955 |
| H | 1.147625744149 | 5.026758105775 | 3.081157305079 |
| H | -0.446553326603 | 1.875813314642 | 3.534855635539 |
| H | -0.947027603386 | 3.521029081068 | 3.881968850454 |
| N | 1.377088738341 | -0.141990340719 | 4.618627432115 |
| H | 0.388645148965 | -0.317954354491 | 4.704001767783 |
| H | 1.664400506488 | 0.687255025155 | 5.123816727271 |
| H | 1.923694719403 | -0.953072052444 | 4.854657697108 |
| H | 0.429237876451 | 1.270419895825 | 1.176850903272 |
| C | -0.529430234494 | 1.637823592253 | 0.809906421966 |
| N | -1.544591664313 | 2.062901207975 | 0.482148325905 |

(Table 3)

|   | x | y | Z |
|---|---|---|---|
| O | 2.765037226263 | -1.231031948409 | 3.380213016379 |
| H | 1.845770895868 | -1.546062814698 | 3.453087981729 |
| H | 3.187791273905 | -1.135593556277 | 4.233233624338 |
| C | 2.998818265012 | -4.516521976419 | 1.789183998093 |
| O | 0.496581592041 | -2.612613474029 | 2.956014171165 |
| C | 1.930564870801 | -5.563669804027 | 1.451144649131 |
| C | 3.214785072661 | -4.444783610384 | 3.308247102363 |
| H | 1.739982520613 | -5.586488914449 | 0.376658029272 |
| H | 3.970783686223 | -3.700521244101 | 3.552621974859 |
| H | 1.009795837361 | -5.288954403294 | 1.966976750274 |
| H | 2.240261040995 | -6.557126556336 | 1.775350258511 |
| H | 3.533468480258 | -5.415969067404 | 3.686225152260 |
| H | 2.278398153246 | -4.158404456720 | 3.784284349778 |
| N | 2.540436370999 | -3.215145043316 | 1.303448918217 |
| H | 2.471815042165 | -3.148041416664 | 0.302363095274 |
| H | 3.013241045960 | -2.421877231233 | 1.719979928534 |
| H | 1.057312380433 | -2.893732575558 | 2.192719277450 |
| H | -0.441401478393 | -2.584658666559 | 2.774114621571 |
| C | 4.282387018287 | -4.888404969664 | 1.136766274508 |
| N | 5.272659925334 | -5.155143932451 | 0.619930717268 |

In Table 1, $N^{gradient}$, $N^{Hessian}$, $N^{EQ}$, and $N^{path}$ respectively correspond to the number of gradient calculations performed until obtaining the product, the number of Hessian calculations (calculations related to second-order differentiation), the number of EQs obtained until obtaining the product, and the number of paths. Here, it was confirmed that a reaction mechanism in which amino nitrile was generated through nucleophilic addition of cyanide anion to iminium ion was obtained in all computations.

As shown in Table 1, the computational cost was highest when a fragment pair was randomly selected. In other words, it was confirmed that the present teaching was effective. Among $\zeta^{Second}$, $\zeta^{Third}$, $\zeta^{Scaled}$, and $\zeta^{Average}$, $\zeta^{Average}$ showed the best performance. This seems because both a path of local thermal equilibration and a path of a slow process corresponding to rare events were efficiently sampled. In a state where reactant molecules weakly associate, there are numerous paths in which the orientation of the molecules can change with a very low barrier of about 10 kJ/mol or less.

Such paths can be efficiently searched by using $\zeta^{Scaled}$ as an index. On the other hand, a path in which a water molecules are released from hemiaminal intermediates and iminium ions are generated has a relatively high barrier of about 100 kJ/mol, and when $\zeta^{Scaled}$ is used as an index, the computation is disadvantageously postponed. On the other hand, when using $\zeta^{Average}$ which is a geometric mean of $\zeta^{Scaled}$ and $\zeta^{Third}$ as an index, both a fragment pair with small $\zeta^{Scaled}$ and a fragment pair with small $\zeta^{Third}$ are searched for. Therefore, it is considered that search was preferentially conducted for a rearrangement path involving a chemical bond with a relatively high barrier, such as conversion from hemiaminal intermediates to iminium ions.

In this example, the target of reaction path search was a system formed of only 20 atoms (N=20) . On the other hand, the number of fragments increases in proportion to the square of N, and hence the cost ratio of random to $\zeta^{Average}$ is also expected to increase rapidly with N. In this example, numerical verification was performed using an organic synthesis reaction as an example. However, in processes that do not involve rearrangement of chemical bonds, such as a conformational change in polymer or a phase transition in an aggregate structure, it is expected that only a path with a low barrier should be preferably searched for by using $\zeta^{Scaled}$ as an index. Therefore, it is recommended that the user uses $\zeta^{Average}$ or $\zeta^{Scaled}$ according to the objective.

[Details of RCMC Method]

The following will detail the RCMC method. Assume that a set of states in the n-th contraction is $N^{(n)}$, a rate constant from a state i to a state j is $k_{i \to j}^{(n)}$, and a Boltzmann distribution in the state i is $P_i^{(n)}$. To put it differently, $N^{(0)}$ is equivalent to a set of all stable structures (EQs) in a reaction path network, $k_{i \to j}^{(0)}$ is equivalent to a rate constant of transition from a stable structure i to a stable structure j, and $P_i^{(n)}$ is equivalent to a Boltzmann distribution of the stable structure i. The contraction operation using these quantities is as follows.

1. The upper limit $k_{MAX}(=1/t_{MAX})$ of a rate constant is given, and n is set at 0. 2. $k_{i \to j}^{(0)}$ is calculated for all elementary processes and a rate constant matrix is obtained. At this stage, when there are two or more elementary processes directly connecting the stable structures i and j, $k_{i \to j}^{(0)}$ is the sum of rate constants for them. When there is no elementary process directly connecting the stable structures i and j, $k_{i-j}^{(0)}$ is set at zero. $k_{i \to i}^{(0)}$ regarding an elementary process connecting stable structures i and i is also set at zero. 3. A Boltzmann distribution $P_i^{(0)}$ is calculated for all stable structures. 4. A (initial) distribution $Q_i^{(0)}$ of all stable structures is given. 5. $\chi_{ii}^{(0)}$ is set at 1 and $\chi_{ji}^{(0)}$ is set at 0($j \neq i$). The following steps 6 to 14 are repeatedly executed as a loop. 6. From all pairs of i and j, a pair with the maximum $k_{i \to j}^{(n)}$ is searched for. 7. If $k_{i \to j}^{(n)} < k_{MAX}$ is satified in the maximum $k_{i \to j}^{(n)}$ obtained in the step 6, the loop is finished. 8. A constant-state approximation is applied to the state i. At this stage, all the rate constants in the rate constant matrix are corrected by using equations 6 and 7.

(Equation 6)

$$k_{k \to l}^{\prime (n+1)} = k_{k \to l}^{(n)} + k_{k \to i}^{(n)} k_{i \to l}^{(n)} \sigma_i^{(n)}$$

(Equation 7)

$$\sigma_i^{(n)} = \frac{1}{\sum_{m \in N^{(n)}} k_{i \to m}^{(n)}}$$

9. Rate constants related to the state i are all set at zero (($k_{i \to m}^{\prime (n+1)} = k_{m \to i}^{\prime (n+1)} = 0$). 10. As shown in an equation 8, the Boltzmann distribution of the state i is set at zero ($P_i^{(n+1)} = 0$), and distribution to another state k($k \neq i$) is performed with a rate constant weight.

(Equation 8)

$$P_k^{(n+1)} = P_k^{(n)} + k_{i \to k}^{(n)} \sigma_i^{(n)} P_i^{(n)}$$

11. As shown in an equation 9, the distribution of the state i is set at zero ($Q_i^{(n+1)} = 0$), and distribution to another state k($k \neq i$) with a rate constant weight is performed.

(Equation 9)

$$Q_k^{(n+1)} = Q_k^{(n)} + k_{i \to k}^{(n)} \sigma_i^{(n)} Q_i^{(n)}$$

12. Contribution of a stable structure j to a state k is updated by using an equation 10.

(Equation 10)

$$\chi_{kj}^{(n+1)} = \chi_{kj}^{(n)} + k_{i \to k}^{(n)} \sigma_i^{(n)} \chi_{ij}^{(n)}$$

13. To reproduce an updated Boltzmann distribution, $k'_{k \to l}^{(n+1)}$ is corrected as shown in an equation 11.

(Equation 11)

$$k_{k \to l}^{(n-1)} = \frac{1}{1 + \sigma_i^{(n)} k_{k \to i}^{(n)}} k_{k \to l}^{\prime (n+1)}$$

14. $N^{(n+1)}$ that is the remainder after the deletion (weighted distribution) of the state i is regarded as a set of the states, and the operation returns to the step 6.

$Q_i^{(0)}$ and $Q_i^{(n)}$ in the above-described steps 4 and 11 are used as $Q^{(m)}_j$ in the equation 3. $\chi_{ii}^{(0)}$, $\chi_{ji}^{(0)}$ (j≠i), and $\chi_{ji}^{(n)}$ in the above-described steps 5 and 12 are used as $\chi_{ji}^{(m)}$ of the equation 3.

<Modifications>

A preferred embodiment of the present teaching has been described above. It should be noted that the present teaching is not limited to the above-mentioned embodiment and various changes, substitutions, and alterations can be made herein without departing from the spirit and scope of the teaching as described in the solution.

For example, in the present embodiment, the rankings is set for each fragment pair so that the smaller the $\zeta$ selected by the user from $\zeta^{Second}$, $\zeta^{Third}$, $\zeta^{Scaled}$, and $\zeta^{Average}$, the higher the ranking. The reaction path search is performed for the fragment pairs in the descending order of the ranking. However, the order of setting the ranking and the order of performing the reaction path search may adopt other approaches. For example, the ranking may be set in two stages for each fragment pair based on whether $\zeta$ is below a certain value or not. After the reaction path search in a random order for fragment pairs that are high in the ranking, the search may be performed in a random order for fragment pairs that are low in the ranking. Also in this case, because the search is performed for the fragment pairs that are high in the ranking prior to the fragment pairs that are low in the ranking, a desired reaction path is more likely to be obtained in advance as compared to cases where the search is executed in a random order for all fragment pairs.

In the above-described embodiment, the state selection unit 40 selects EQi with the largest $v_i$ represented by the above-described equation 5 as the starting point of the reaction path search. In this regard, the starting point of the reaction path search may be selected based on a reference values other than $v_i$. In this case, the starting point of the reaction path search is selected in such a way that an equilibrium state with a large $\Lambda_i$ is likely to be selected, and the larger $n_{all}$ is, the less likely a difference in $\Lambda_i$ is reflected in the likeliness of the selection of the equilibrium state.

[Reference Signs List]

1      reaction-path search system
10      storage unit
20      fragment pair determination unit
30      fragment pair ranking acquisition unit
40      state selection unit
50      reaction path computation unit

**Claims**

1. A reaction-path search program for causing at least one computer to function as a reaction-path search system which is configured to calculate a reaction path in a structure formed of multiple atoms as a change in a structure represented by a positional relationship of the multiple atoms,
the reaction-path search system including:

   a structural change calculation unit which is configured to calculate a change in the structure in which a result of a function $F^{AFIR}(Q)$ of an equation 1 calculated based on an equation 2 is at the minimum, where, in regard to a fragment pair of a fragment A composed of $N_1$ ($N_1$ is a natural number) atoms sampled from the multiple atoms and a fragment B composed of $N_2$ ($N_2$ is a natural number) atoms sampled from the multiple atoms and different from the atoms of the fragment A, potential energy at a geometric parameter Q indicating positions of the multiple atoms is E(Q), distance between s-th (s is a natural number satisfying $s \leq N_1$) atom belonging to the fragment A and a t-th (t is a natural number satisfying $t \leq N_2$) atom belonging to the fragment B is $r_{st}$, $R_s$ and $R_t$ are covenant radii of the s-th atom and the t-th atom, respectively, $\rho$ is either +1 or -1, and p and $\alpha$ are constants;

$$\text{(Equation 1)}$$

$$F^{AFIR}(\boldsymbol{Q}) = E(\boldsymbol{Q}) + \rho\alpha\frac{\sum_{s\in A}\sum_{t\in B}\omega_{st}r_{st}}{\sum_{s\in A}\sum_{t\in B}\omega_{st}}$$

$$\text{(Equation 2)}$$

$$\omega_{st} = \left[\frac{R_s + R_t}{r_{st}}\right]^p$$

   a differential coefficient calculation unit which is configured to calculate, for each of the fragment pairs formed of different combinations of atoms, at least one of a second differential coefficient b or a third differential coefficient a of E(Q) at the positions of the multiple atoms,
   which correspond to a first equilibrium state that is one of equilibrium states where E(Q) takes a local minimum value; and
   an equilibrium state change calculation unit which is configured to cause the structural change calculation unit to calculate the change in the structure in transition from the first equilibrium state to a second equilibrium state that is one of the equilibrium states, prior to the other fragment pairs with lower priority than the fragment pair with higher priority based on the magnitude of a and b calculated by the differential coefficient calculation unit among the fragment pairs.

2. The reaction-path search program according to claim 1, wherein,

   the reaction-path search system further includes a state selector which is configured to select one of N equilibrium states (N is a natural number of 2 or more) formed of all second equilibrium states obtained based on past calculation results by the equilibrium state change calculation unit and an initial state as the first equilibrium state, and
   the equilibrium state change calculation unit repeatedly causes the structural change calculation unit to calculate the change in the structure regarding the fragment pair with respect to the transition from the first equilibrium state selected by the state selector to the second equilibrium state.

3. The reaction-path search program according to claim 2, wherein,

   the reaction-path search system further includes a rate constant contraction unit which is configured to obtain a rate constant matrix, which is formed of 1*1 rate constants regarding transition between 1 (1 is a natural number satisfying 1<N) super states expressed as a weighted sum of the N equilibrium states, from a rate constant matrix of N rows and N columns, which is formed of N*N rate constants regarding transition between the N equilibrium states, by performing contracting of the rate constant matrix m times (m is a natural number satisfying m=N-l) based on an RCMC method, and

the state selector selects one of the N equilibrium states as the first equilibrium state for each of m=1, 2, ..., M (M is a natural number satisfying M<N) based on a result of acquisition of a contracted rate constant matrix by the rate constant contraction unit so that, the larger $p_i(m)$ calculated based on an equation 3 and $\Lambda_i$ calculated based on an equation 4 are, the more likely $EQ_i$ is selected, when each of the N equilibrium states is $EQ_i$ (i is a natural number equal to or smaller than N), each of the 1 super states is $SS_j$ (j is a natural number equal to or smaller than I), population of $EQ_i$ after performing the contraction m times is $p_i(m)$, population of $SS_j$ after performing the contraction m times is $Q_j(m)$, contribution of $EQ_i$ to $SS_j$ after performing the contraction m times is $\chi_{ji}(m)$, relative Gibbs energy of $EQ_i$ is $\Delta G_i$, a gas constant is R, and a model temperature parameter is $T_R$.

(Equation 3)

$$p_i^{(m)} = \sum_j^{\text{all SSs}} Q_j^{(m)} \frac{\chi_{ji}^{(m)} \exp\left[\frac{-\Delta G_i}{RT_R}\right]}{\sum_k^{\text{all EQs}} \chi_{jk}^{(m)} \exp\left[\frac{-\Delta G_k}{RT_R}\right]}$$

(Equation 4)

$$\Lambda_i = \sum_{m=1}^{M} \left| p_i^{(m)} - p_i^{(m-1)} \right|$$

**4.** The reaction-path search program according to claim 3, wherein,

the state selector selects one of the N equilibrium states as the first equilibrium state so that the likeliness of selection of $EQ_i$ increases as $\Lambda_i$ increases, the likeliness of selection of $EQ_i$ increases as the number of times $n_i$ of calculation of the change in the structure by the structural change calculation unit while $EQ_i$ is set as the first equilibrium state decreases, and the larger the total number of times $n_{all}$ of calculation of the change in the structure by the structural change calculation unit for the N equilibrium states is, the less likely a diference in $\Lambda_i$ is reflected in a difference in the likeliness of selection of the equilibrium state.

**5.** The reaction-path search program according to claim 3 or 4, wherein,

when the total number of times of calculation of the change in the structure by the structural change calculation unit for the N equilibrium states is $n_{all}$, the number of times of calculation of the change in the structure by the structural change calculation unit while $EQ_i$ is set as the first equilibrium state is $n_i$, $\xi_i$ is a real number not smaller than 0 and not larger than 1, and each of $\alpha$ and $\beta$ is a real number not smaller than 0 and not larger than 1, the state selector selects $EQ_i$ with which $v_i$ in an equation 5 is the largest, as the first equilibrium state.

(Equation 5)

$$v_i = \xi_i \left( \Lambda_i + \alpha^{\frac{n_i}{\log(n_{all})}} \right) \beta^{\frac{n_i}{\log(n_{all})}}$$

**6.** A system for calculating a reaction path in a structure formed of multiple atoms as a change in a structure represented by a positional relationship of the multiple atoms, the system comprising:

a structural change calculation unit which is configured to calculate a change in the structure in which a result of a function $F^{AFIR}(Q)$ of an equation 1 calculated based on an equation 2 is at the minimum, where, in regard to a fragment pair of a fragment A composed of $N_1$ ($N_1$ is a natural number) atoms sampled from the multiple atoms and a fragment B composed of $N_2$ ($N_2$ is a natural number) atoms sampled from the multiple atoms and different from the atoms of the fragment A, potential energy at a geometric parameter Q indicating positions of the multiple atoms is E(Q), distance between s-th (s is a natural number satisfying $s \leq N_1$) atom belonging to the fragment A and a t-th (t is a natural number satisfying $t \leq N_2$) atom belonging to the fragment B is $r_{st}$, $R_s$ and $R_t$ are covenant radii of the s-th atom and the t-th atom, respectively, $\rho$ is either +1 or -1, and p and $\alpha$ are

constants;

(Equation 1)

$$F^{\mathrm{AFIR}}(\boldsymbol{Q}) = E(\boldsymbol{Q}) + \rho\alpha\frac{\sum_{s\in A}\sum_{t\in B}\omega_{st}r_{st}}{\sum_{s\in A}\sum_{t\in B}\omega_{st}}$$

(Equation 2)

$$\omega_{st} = \left[\frac{R_s + R_t}{r_{st}}\right]^p$$

a differential coefficient calculation unit which is configured to calculate, for each of the fragment pairs formed of different combinations of atoms, at least one of a second differential coefficient b or a third differential coefficient a of E(Q) at the positions of the multiple atoms,
which correspond to a first equilibrium state that is one of equilibrium states where E(Q) takes a local minimum value; and
an equilibrium state change calculation unit which is configured to cause the structural change calculation unit to calculate the change in the structure in transition from the first equilibrium state to a second equilibrium state that is another one of the equilibrium states, prior to the other fragment pairs with lower priority than the fragment pair with higher priority based on the magnitude of a and b calculated by the differential coefficient calculation unit among the fragment pairs.

7. A method for calculating a reaction path in a structure formed of multiple atoms as a change in a structure represented by a positional relationship of the multiple atoms, the method comprising:

a structural change calculation step of calculating a change in the structure in which a result of a function $F^{\mathrm{AFIR}}(Q)$ of an equation +1 calculated based on an equation 2 is at the minimum, where, in regard to a fragment pair of a fragment A composed of $N_1$ ($N_1$ is a natural number) atoms sampled from the multiple atoms and a fragment B composed of $N_2$ ($N_2$ is a natural number) atoms sampled from the multiple atoms and different from the atoms of the fragment A, potential energy at a geometric parameter Q indicating positions of the multiple atoms is E(Q), distance between s-th (s is a natural number satisfying $s\leq N_1$) atom belonging to the fragment A and a t-th (t is a natural number satisfying $t\leq N_2$) atom belonging to the fragment B is $r_{st}$, $R_s$ and $R_t$ are covenant radii of the s-th atom and the t-th atom, respectively, $\rho$ is either +1 or -1, and p and $\alpha$ are constants;

(Equation 1)

$$F^{\mathrm{AFIR}}(\boldsymbol{Q}) = E(\boldsymbol{Q}) + \rho\alpha\frac{\sum_{s\in A}\sum_{t\in B}\omega_{st}r_{st}}{\sum_{s\in A}\sum_{t\in B}\omega_{st}}$$

(Equation 2)

$$\omega_{st} = \left[\frac{R_s + R_t}{r_{st}}\right]^p$$

a differential coefficient calculation step of calculating,
for each of the fragment pairs formed of different combinations of atoms, at least one of a second differential coefficient b or a third differential coefficient a of E(Q) at the positions of the multiple atoms, which correspond to a first equilibrium state that is one of equilibrium states where E(Q) takes a local minimum value; and
an equilibrium state change calculation step of calculating,
by the structural change calculation step, the change in the structure in transition from the first equilibrium state to a second equilibrium state that is another one of the equilibrium states, prior to the other fragment pairs with lower priority than the fragment pair with higher priority based on the magnitude of a and b calculated by the differential coefficient calculation unit among the fragment pairs.

FIG.1

FRAGMENT A

FRAGMENT B

SECOND LAYER

FIRST LAYER

FIRST LAYER

SECOND LAYER

FRAGMENT PAIR

0-TH LAYER

D

0-TH LAYER

SECOND LAYER

FIRST LAYER

FIRST LAYER

SECOND LAYER

FIG.2

REACTION PATH

EQ₄ EQ₆ EQ₂ EQ₁ EQ₅ EQ₃

FIG.3

STORAGE UNIT ～10

FRAGMENT PAIR DETERMINATION UNIT ～20

FRAGMENT PAIR RANKING ACQUISITION UNIT ～30

STATE SELECTION UNIT ～40

REACTION PATH COMPUTATION UNIT ～50

FIG.4

## FIG.5

```
START
```

↓

OBTAIN RANKING OF FRAGMENT PAIR
FOR INITIAL STATE $EQ_1$ — S1

↓

PERFORM REACTION PATH SEARCH STARTING FOR FRAGMENT PAIR
CORRESPONDING TO THE HIGHEST RANKING → OBTAIN $EQ_2$ — S2

↓

CALCULATE $\nu_i$ FOR EACH OBTAINED $EQ_i$ ( i = 1, 2, ··· ) — S3

↓

HAS RANKING OF FRAGMENT
PAIR ALREADY BEEN OBTAINED FOR $EQ_i$
CORRESPONDING TO LARGEST $\nu_i$? — S4

Yes → / No ↓

ACQUIRE RANKING OF FRAGMENT PAIR FOR $EQ_i$
CORRESPONDING TO LARGEST $\nu_i$ — S5

↓

FOR $EQ_i$ CORRESPONDING TO LARGEST $\nu_i$, PERFORM REACTION
PATH SEARCH FOR FRAGMENT PAIR WITH HIGHEST RANKING
AMONG FRAGMENT PAIRS FOR WHICH REACTION PATH
SEARCH HAS NOT YET BEEN EXECUTED → OBTAIN NEW $EQ_i$ — S6

↓

IS SEARCH CONDITION SATISFIED? — S7

No → / Yes ↓

```
END
```

## INTERNATIONAL SEARCH REPORT

| International application No. |
| --- |
| **PCT/JP2023/002443** |

**A. CLASSIFICATION OF SUBJECT MATTER**

*G16C 20/10*(2019.01)i
FI: G16C20/10

According to International Patent Classification (IPC) or to both national classification and IPC

**B. FIELDS SEARCHED**

Minimum documentation searched (classification system followed by classification symbols)

G16C20/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2008-52308 A (UNIV. WASEDA) 06 March 2008 (2008-03-06) entire text, all drawings | 1-7 |
| A | JP 2021-82066 A (SUMITOMO RUBBER IND., LTD.) 27 May 2021 (2021-05-27) entire text, all drawings | 1-7 |
| A | JP 2020-129270 A (FUJITSU LTD.) 27 August 2020 (2020-08-27) entire text, all drawings | 1-7 |
| A | WO 2016/052662 A1 (OSAKA UNIV.) 07 April 2016 (2016-04-07) entire text, all drawings | 1-7 |

☐ Further documents are listed in the continuation of Box C.  ☑ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
| --- | --- |
| **03 March 2023** | **14 March 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/002443**

| Patent document cited in search report | | | Publication date (day/month/year) | Patent family member(s) | | | Publication date (day/month/year) |
|---|---|---|---|---|---|---|---|
| JP | 2008-52308 | A | 06 March 2008 | WO 2006/062095 A1 entire text, all drawings | | | |
| JP | 2021-82066 | A | 27 May 2021 | (Family: none) | | | |
| JP | 2020-129270 | A | 27 August 2020 | US 2020/0258594 A1 whole document | | | |
| WO | 2016/052662 | A1 | 07 April 2016 | US 2017/0220717 A1 whole document EP 3203401 A1 CA 2962730 A1 CN 107004064 A | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SATOSHI MAEDA ; YU HARABUCHI**. Exploring paths of chemical transformations in molecular and periodic systems: An approach utilizing force. *Wiley Interdisciplinary Reviews: Computational Molecular Science*, vol. 11, e1538

- *J. Chem. Phys.*, 1991, vol. 94, 751
- *J. Comput. Chem.*, 2014, vol. 35, 166
- *Chem. Lett.*, 2019, vol. 48, 47